Europäisches Patentamt

(19) European Patent Office

Office européen des brevets

(11) Publication number: **0 458 486 A1**

(12) **EUROPEAN PATENT APPLICATION**

(21) Application number: **91304105.9**

(22) Date of filing: **07.05.91**

(51) Int. Cl.⁵: **A61B 17/60, F16C 11/04**

(30) Priority: **04.05.90 GB 9010180**

(43) Date of publication of application:
**27.11.91 Bulletin 91/48**

(84) Designated Contracting States:
**CH DE FR GB IT LI SE**

(71) Applicant: **Richardson, James Bruce**
**3 Victoria Road**
**Oswestry, Shropshire SY11 2HT (GB)**

(72) Inventor: **Richardson, James Bruce**
**3 Victoria Road**
**Oswestry, Shropshire SY11 2HT (GB)**

(74) Representative: **Murgitroyd, Ian G. et al**
**Ian G. Murgitroyd and Company Mitchell**
**House 333 Bath Street**
**Glasgow G2 4ER (GB)**

(54) **Bone fixator.**

(57) A mechanism having a particular application as a bone fixator for the fixation of two substantially co-linear members (A, B), which enables the controlled pivotal motion of the two members (2, 3) in two axes. The mechanism includes a dual axis hinge (1) having two members (2, 3) that are pivotally connectable by engageable pivots (6, 7) at each end of the members (2, 3), such that the members (2, 3) may pivot with respect to each other when one of the engageable pivots is engaged and the other pivot is released. The mechanism also includes an attachable elongate column (8) having a plurality of clamps (9), to each of which is affixed a bar (10) holding a further clamp (11) which affixes a bone screw (12). The bone screws (12) are attached to different portions of the substantially co-linear members (A, B), which may be, for example, a broken bone. The mechanism may also be used to encourage free and controlled cyclic bending centred on each pivot (6, 7) when the clamps (9, 11) at either end of each of the bars (10) are released.

EP 0 458 486 A1

Fig.3

This invention relates to a bone fixator.

The production of callus is an important step in the healing process for broken or severely fractured bones. However, callus is not generally produced unless the healing tissues are stimulated by movement. Movement of one portion of a broken bone with respect to the adjacent portion of the bone will result in an increased production of callus along the line of contact between the two bone portions.

A cyclic movement of one bone portion with respect to the other will produce the most uniform growth of callus, however cyclic motion has certain disadvantages not least that it is difficult to generate. A bending or pivotal motion would be preferable to the cyclic motion, but would have the disadvantage of a non-uniform distribution of movement which would result in a non-uniform production of callus. Therefore, a weak area would be produced on either side of the line of movement of the bone portions, where less callus is produced.

The introduction of the bone fixator which allows controlled pivotal movement of bone portions with respect to each other, wherein the pivotal axis may be moved radially with respect to the bone portions to allow uniform pivotal movement at points around the bone and thus a uniform distribution of callus growth would constitute a considerable improvement. Such an invention would be of increased value if the axes of pivotal motion lie in the same plane as the interface of the break between the two bone portions.

Controlled pivotal movement of bone portions would also allow measurement of fracture stiffness without the requirement of removal of the bone fixator.

According to a first aspect of the present invention there is provided a mechanism for the fixation of two substantially collinear members which enables the pivotal motion of the two members in two axes.

Preferably, the two pivot axes lie in a plane which includes the interface between the two members.

Preferably, the mechanism is an external bone fixator.

Preferably, the angle between the two axes is approximately 90 degrees.

According to a second aspect of the present invention there is provided a dual axis hinge comprising two members which are arranged such that a first end of each of the members lies in a first plane and a second end of each of the members lies in a second plane, the first and second planes being at an angle to each other, the members being pivotally connectable by a first engagable pivot in the first plane and a second engagable pivot in the second plane, such that the members may pivot with respect to each other on an axis orthogonal to either of the planes when the pivot in that plane is engaged and the pivot in the other plane is released.

Preferably, a dual axis hinge also comprises a first spring and second spring, the first spring being located at the first engagable pivot and the second spring being located at the second engagable pivot.

Preferably, the members are curved blades.

Preferably, the angle between the first and second planes is approximately 90 degrees.

Preferably, each member is substantially L-shaped having a first portion in the form of a curved blade and a second portion orthogonal to the first portion, in the form of a flat blade.

Preferably, the members are positioned such that the second portion of each member lies in the first plane, one member having its second portion directed upwards of the first portion and the other member having its second portion directed downwards of the first portion.

Preferably, each member includes a means of attachment, to a further support member, affixed to the second portion of the member.

Preferably, the dual axis hinge is made from a light weight material which does not provide hindrance to radiography of the fracture.

According to a third aspect of the present invention there is provided a mechanism for the fixation of two substantially co-linear members according to the first aspect of the present invention and including a dual axis hinge in accordance with the second aspect of the present invention.

Preferably, the mechanism is an external bone fixator.

Preferably, the mechanism is made from a light weight material that does not provide hindrance to radiography of the fracture.

Preferably, the second portion of each of the hinge members includes an attachable elongate column, each of the columns having a plurality of clamps each of which affixes a bar, in a plane substantially orthogonal to the column and parallel to the first portion of the hinge member, each bar having a further clamp which affixes a bone screw to the bar.

Preferably, the clamps enable the attachment of two elongate members in substantially orthogonal planes by the movement of one screw, thus increasing the ease of use of the bone fixator.

Preferably, the bone screws attached to each of the hinge members, via the rods and column, are attached to different portions of a broken bone, when in use.

Preferably, the two portions of the broken bone are pivotally movable with respect to each other, in a plane containing the interface between the portions, under the control of the fixator, when in use.

Preferably, the bone portions are pivotally movable with respect to each other in two axis, which are perpendicular to the first and second planes of the hinge members, respectively, while attached to the dual axis hinge.

Preferably, force applied to the bone through the movement of the limb containing the bone results in the controlled pivotal movement of the fixator, which results in the controlled pivotal movement of the bone about the interface.

Alternatively, force applied to the fixator results in the controlled pivotal movement of the fixator and the portions of the bone, the bone pivoting about the interface.

When the fixator is securely attached to the portions of the bone the only movement of the portions of the bone which is possible is the pivotal movement in either of the pivotal axes, perpendicular to the first or second planes of the dual axis hinge.

If the clamps at either end of each of the rods are released, the portions of bone are no longer supported by the fixator and the portions of the bone may be moved cyclically with respect to each other.

Preferably, the inclusion of springs wound around the first engagable pivot and second engagable pivot encourage free and controlled cyclic bending centred on each pivot, when the clamps at either end of each of the rods are released.

Preferably, when the fixator is securely attached to the bone, the bone may be pivotally moved in one of two axis which are perpendicular to the first and second plane of the dual axis hinge, the radial position of the axes with respect to the bone being alterable by altering the radial position of the first and second engagable pivots on the hinge with respect to the bone.

Most preferably, the radial position of the first and second engagable pivots are altered by altering the separation between the clamps on the fixator rods, by loosening the clamps on one end of the rods, adjusting the separation and re-tightening the clamps.

Thus the fixator enables controllable pivotal movement of two portions of a bone in either of two known axes, the axes being at any pre-set radial position with respect to the bone.

Preferably, the longitudinal force applied on the bones may be altered by releasing the clamps attached to the column, altering the force applied to the bone and retightening the clamps.

An embodiment of the present invention will now be described, by way of example, with reference to the accompanying drawings in which:-

Fig. 1 is a perspective view of a dual axis hinge, in accordance with the second aspect of the present invention, pivoting in the first plane;

Fig. 2 is a perspective view of the dual axis hinge of Fig. 1, pivoting in the second plane; and

Fig. 3 is a perspective view of a bone fixator in accordance with the present invention.

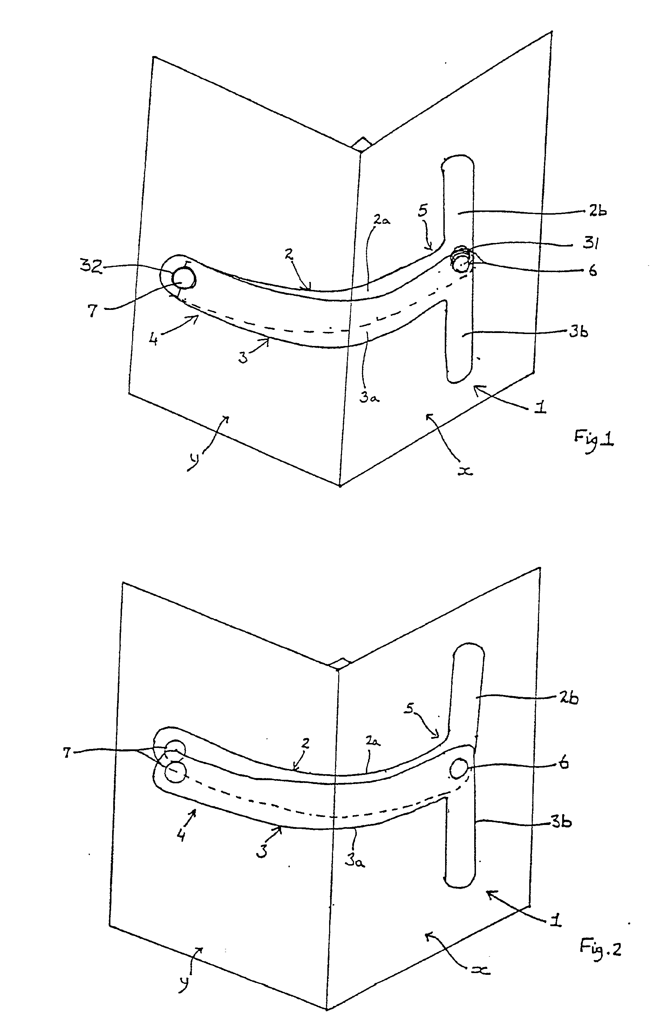

With reference to the accompanying drawings, Fig. 1 and Fig. 2 illustrate a dual axis hinge 1 comprising two members 2 and 3 which are arranged such that the first portion 4 of the members 2 and 3 lie in the first plane X and the second ends 5 of each of the members 2 and 3 lie in a second plane Y, the first and second planes X and Y being at an angle to each other, the members being pivotally connectable by a first engagable pivot 6 in the first plane X and a second engagable pivot 7 in the second plane Y such that the members 2 and 3 may pivot with respect to each other on an axis orthogonal to either plane X or plane Y when the pivot 6 or 7 in that plane is engaged and the pivot 7 or 6 in the other plane is released.

A first spring 31 is wound around the first engagable pivot 6 in the first plane X and a second spring 32 is similarly wound around the second engagable pivot 7 in the second plane Y. The springs 31, 32 prevent jamming of the members 2, 3 at the first and second engagable pivots 6, 7 and encourage free and controlled cyclic bending centred at each pivot. The spring wire rotates around the pivots as weight on each member 2, 3 is applied. The turning of the spring wire enables the spring to also act as a hinge when the load is applied, for example, when walking in the case of a fracture in a leg bone, and the spring also acts to return the hinge 1 to its pre-set equilibrium position.

The members 2 and 3 are curved blades and the angle between the first and second planes X and Y is approximately 90 degrees.

Each member 2 and 3 is substantially L-shaped having a first portion 2a and 3a in a form of a curved blade and the second portion 2b and 3b, orthogonal to the first portion 2a and 3a and in the form of a flat blade.

The members 2 and 3 are positioned such that the second portions 2b and 3b of each member lies in the first plane X, one member 2 having its second portion 2b directed upwards of its first portion 2a and the other member 3 having its second portion 3b directed downwards of its first portion 3a.

Fig. 1 illustrates a slight pivotal movement of the blades 2 and 3 in the second plane Y when the first engagable pivot 6 is released and the second engagable pivot 7 is engaged.

Fig. 2 illustrates a slight pivotal motion in the first plane X when the first engagable pivot 6 is engaged and the second engagable pivot 7 is released.

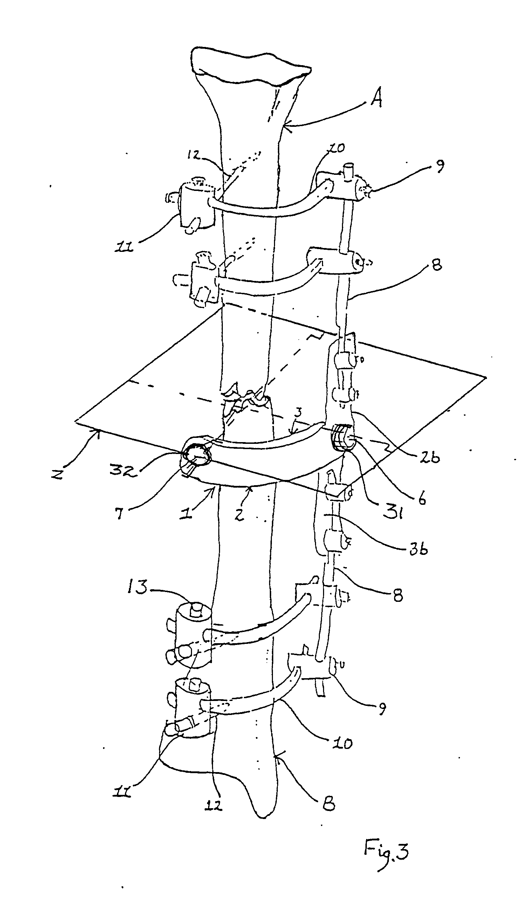

Fig. 3 shows a bone fixator comprising a dual axis hinge 1, as illustrated in Figs. 1 and 2, two elongate columns 8 each of which is attached to the second portions 2b and 3b of each of the hinge members 2 and 3. Each of the columns 8 have a plurality of clamps 9 each of which affixes a bar 10 in a plane substantially orthogonal to the column 8 and parallel to the first portion 2a and 3a of the hinge member 2 or 3. The end of each bar 10, away from the columns 8, has a further clamp 11 which affixes a bone screw 12 to the rod 10.

The clamps 9 and 11 enable the attachment of two elongate members in orthogonal planes by the movement of one screw 13, thus increasing the ease of use of the bone fixator. The clamp design allows simultaneous locking of two orthogonal bars with a single bolt in compression, with cylindrically shaped surfaces to allow compression over relatively large surface areas and thus achieve greater strength when clamped.

The bone screws 12 attached to each of the hinge members 2 and 3, via the rods 10 and columns 8, are attached to different portions A and B of a broken limb, when in use.

The two portions A and B of the broken bone are pivotally movable with respect to each other, in a plane Z containing the interface between the portions A and B, under the control of the fixator, when in use.

The bone portions A and B are pivotally movable with respect to each other in two axes, while attached to the dual axis hinge 1, the axes being perpendicular to the first and second planes, X and Y respectively, of the hinge. This movement may result from force applied to the bone through the movement of the limb containing the bone, the direction of movement being controlled by the dual axis hinge 1.

Alternatively, force may be applied to the fixator resulting in the controlled pivotal movement of the fixator and the portions of the bone. In both cases the bone portions A and B are pivoted in the plane Z containing the interface between the two bone portions A and B.

If a cyclic rotation of the portions A and B of the bone with respect to each other is required then the clamps 9 or 11 at either end of the rods 10 may be released, releasing the fixator and freeing the bone portions A and B to be moved cyclically with respect to each other.

In order to ensure an equal distribution of callus growth around the interface between the two bones, the radial position of the pivotal axis may be altered with respect to the bone by altering the position of the first and second engagable pivots 6 and 7 with respect to the bone. The radial position of the pivots 6 and 7 are altered by altering the separation between the clamps 9 and 11 on the fixator rods 10, by loosening the clamps 9 or 11 on one end of the rods 10, adjusting the separation between the clamps 9 and 11 and retightening them to the rods 10.

Thus the fixator enables controllable pivotal movement of two bone portions A and B in either of two known axes, the axes being located in any pre-set radial position with respect to the bone, the pivotal movement being in a plane Z containing the interface between the bones.

The bone fixator may be made of a light weight material such as plastics or composites, including glass fibre of carbon fibre. Materials of this type are preferable as they have high strength to weight characteristics and allow easy radiography of the fractured site.

Another advantage of a bone fixator in accordance with the invention is that it by a controlled bending of the fixator measurements of fracture stiffness may be made without removing the fixator.

The invention also provides considerable advantages over that which has been known previously in that they two plane bending has application in external fixation across a joint as it allows movement and thus reduces side effects such as stiffness in the joint. The invention also enables movement in two planes without shortening of the fracture which has been a significant problem in the past, particularly in relation to fractures located in the proximity of a ball joint.

The longitudinal force applied to the bone portions A and B may be altered by releasing the clamps 9 on the column 8, altering the force applied to the bone portions A and B and retightening the clamps 9.

Modifications and improvements may be incorporated without departing from the scope of the invention.

## Claims

1. A mechanism for the fixation of two substantially co-linear members characterised in that it enables the pivotal motion of the two members in two axes.

2. A mechanism as claimed in Claim 1, wherein the two pivot axes lie in a plane which includes the interface between the two members.

3. A mechanism as claimed in Claim 1 or Claim 2, wherein the mechanism is an external bone fixator.

4. A mechanism as claimed in any one of the preceding Claims, wherein the angle between the two axes is approximately 90 degrees.

5. A dual axis hinge (1) comprising two members (2, 3) which are arranged such that a first end of each of the members lies in a first plane (x) and a second end of each of the members lies in a second plane (y), the first and second planes (x, y) being at an angle to each other, characterised in that the members are pivotally connectable by a first engagable pivot (6) in the first plane (x) and a second engagable pivot (7) in the second plane (y), such that the members (2, 3) may pivot with respect to each other on an axis orthogonal to either of the planes (x, y) when the pivot in that plane is engaged and the pivot in the other plane is released.

6. A dual axis hinge (1) as claimed in Claim 5 comprising a first spring (31) and second spring (32), the first spring (31) being located at the first engagable pivot (6) and the second spring (32) being located at the second engagable pivot (7).

7. A hinge (1) as claimed in Claim 5 or Claim 6, wherein the members (2, 3) are curved blades.

8. A hinge (1) as claimed in any one of Claims 5 to 7, wherein the angle between the first and second planes (x, y) is approximately 90 degrees.

9. A hinge (1) as claimed in any one of Claims 5 to 8, wherein each member (2, 3) is substantially L-shaped having a first portion (2a, 3a) in the form of a curved blade and a second portion (2b, 3b) orthogonal to the first portion (2a, 3a), in the form of a flat blade.

10. A hinge (1) as claimed in any one of Claims 5 to 9, wherein the members (2, 3) are positioned such that the second portion (2b, 3b) of each member (2, 3) lies in the first plane (x), one member having its second portion directed upwards of the first portion and the other member having its second portion directed downwards of the first portion.

11. A hinge (1) as claimed in any one of Claims 5 to 10, wherein each member (2, 3) includes a means of attachment, to a further support member (8), affixed to the second portion (2b, 3b) of the member (2, 3).

12. A hinge (1) as claimed in any one of Claims 5 to 11, made from a light weight material which does not provide hindrance to radiography of the fracture.

13. A mechanism for the fixation of two substantially co-linear members (A, B) as claimed in any one of Claims 1 to 4 and including a dual axis hinge (1) as claimed in any one of Claims 5 to 12.

14. A mechanism as claimed in Claim 13, wherein the mechanism is an external bone fixator.

15. A mechanism as claimed in Claim 14 made from a light weight material that does not provide hindrance to radiography of the fracture.

16. A mechanism as claimed in Claim 14 or Claim 15, wherein the second portion (26, 36) of each of the hinge members (2, 3) includes an attachable elongate column (8), each of the columns (8) having a plurality of clamps (9) each of which affixes a bar (10), in a plane substantially orthogonal to the column (8) and parallel to the first portion (2a, 2b) of the hinge member (2, 3), each bar (10) having a further clamp (11) which affixes a bone screw (12) to the bar (10).

17. A mechanism as claimed in Claim 16, wherein the clamps enable the attachment of two elongate members in substantially orthogonal planes by the movement of one screw, thus increasing the ease of use of the bone fixator.

18. A mechanism as claimed in Claim 16 or Claim 17, wherein the bone screws (12) attached to each of the hinge members (2, 3), via the bars (10) and column (8), are attached to different portions (A, B) of a broken bone, when in use.

19. A mechanism as claimed in Claim 18, wherein the two portions (A, B) of the broken bone are pivotally movable with respect to each other, in a plane (2) containing the interface between the portions (A, B), under the control of the fixator, when in use.

20. A mechanism as claimed in any one of Claims 16 to 19, wherein the inclusion of springs (31, 32) wound around the first engagable pivot (6) and second engagable pivot (7) encourage free and controlled cyclic bending centred on each pivot (6, 7), when the clamps (9, 11) at either end of each of the bars (10) are released.

21. A mechanism as claimed in any one of Claims 16 to 19, wherein the bone portions (A, B) are pivotally movable with respect to each other in two axes, which are perpendicular to the first (x) and second (y) planes of the hinge members (2, 3), respectively, while attached to the dual axis hinge (1).

22. A mechanism as claimed in any one of Claims 14 to 21, wherein force applied to the bone through the movement of the limb containing the bone results in the controlled pivotal movement of the fixator, which results in the controlled pivotal movement of the bone about the interface.

23. A mechanism as claimed in any one of Claims 14 to 21, wherein force applied to the fixator results in the controlled pivotal movement of the fixator and the portions (A, B) of the bone, the bone pivoting about the interface.

24. A mechanism as claimed in any one of Claims 14 to 23, wherein when the fixator is securely attached to the bone, the bone may be pivotally moved in one of two axes which are perpendicular to the first and second plane (x, y) of the dual axis hinge (1), the radial position of the axes with respect to the bone being alterable by altering the radial position of the first and second engagable pivots (6, 7) on the hinge (1) with respect to the bone.

25. A mechanism as claimed in Claim 24, wherein the radial position of the first and second engagable pivots (6, 7) are altered by altering the separation between the clamps (9, 11) on the fixator bars (10), by loosening the clamps on one end of the bars (10), adjusting the separation and re-tightening the clamps.

26. A mechanism as claimed in any one of Claims 14 to 25, wherein the longitudinal force applied on the bone may be altered by releasing the clamps (9) attached to the column (8), altering the force applied to the bone and retightening the clamps (9).

Fig 1

Fig. 2

Fig.3

**PARTIAL EUROPEAN SEARCH REPORT**
which under Rule 45 of the European Patent Convention shall be considered, for the purposes of subsequent proceedings, as the European search report

European Patent Office

Application number

EP 91 30 4105

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 215 392 (HARDWARE & SYSTEMS PATENTS) <br><br> * Figures 2-3 * | <br><br> 5,7-8 | A 61 B 17/60 <br> F 16 C 11/04 |
| A | FR-A-1 231 075 (J.-F.-G. ALGAYRES) <br><br> * Figures 2-6 * | <br><br> 5 | |
| A | EP-A-0 177 270 (UNIVERSITY COLLEGE LONDON) <br><br> * Abstract; figure 1 * | | |
| A | FR-A-2 576 774 (ISSOIRE AVIATION) <br><br> * Abstract; figure 1 * | | TECHNICAL FIELDS SEARCHED (Int. Cl.4) <br><br> A 61 B <br> E 05 D <br> F 16 C |

----

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 5-26

Claims searched incompletely:

Claims not searched: 1-4

Reason for the limitation of the search:

Claims 1-4 contravene art. 84 & Rule 29.1 EPC. These claims attempt to define the invention by a result to be achieved.

| Place of search | Date of completion of the search | Examiner |
|---|---|---|
| THE HAGUE | 02-08-1991 | NICE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1.03.82